# EUROPÄISCHE PATENTSCHRIFT

(11) **EP 2 810 697 B1**
(45) Veröffentlichungstag und Bekanntmachung des Hinweises auf die Patenterteilung: **06.07.2016**
(21) Anmeldenummer: 14153647.4
(22) Anmeldetag: 03.02.2014
(51) Int. Cl.: A61Q 7/00, A61Q 5/00, A61K 8/97

(54) **Haarwuchsmittel enthaltend Leontopodium Extrakt**
Hair-growth agent containing Leontopodium extract
Produit pour la croissance des cheveux contenant de l'extrait de Leontopodium

(30) Priorität: 05.06.2013 DE 102013210463
(43) Veröffentlichungstag der Anmeldung: 10.12.2014
(73) Patentinhaber: Henkel AG & Co. KGaA, 40589 Düsseldorf (DE)
(72) Erfinder: Fuhrmann, Guido, 41812 Erkelenz (DE); Giesen, Melanie, 47608 Geldern (DE); Miehlich, Kristin, 42119 Wuppertal (DE)

(56) Entgegenhaltungen:
- WO-A1-2013/180526
- DATABASE GNPD [Online] MINTEL; Juli 2009 (2009-07), Aekyung: "Enhanced-Elasticity Revitalizing Shampoo", XP002730628, Database accession no. 1138706
- DATABASE GNPD MINTEL; Oktober 2011 (2011-10), Bene Cosmetics: "Damage Care Mask", XP002730629, Database accession no. 1651341
- MELANIE GIESEN ET AL: "Ageing processes influence keratin and KAP expression in human hair follicles", EXPERIMENTAL DERMATOLOGY, Bd. 20, Nr. 9, 1. September 2011 (2011-09-01), Seiten 759-761, XP055033560, ISSN: 0906-6705, DOI: 10.1111/j.1600-0625.2011.01301.x
- SUBRAMANYA R D ET AL: "Transcriptional profiling in alopecia areata defines immune and cell cycle control related genes within disease-specific signatures", GENOMICS, ACADEMIC PRESS, SAN DIEGO, US, Bd. 96, Nr. 3, 1. September 2010 (2010-09-01), Seiten 146-153, XP027213273, ISSN: 0888-7543 [gefunden am 2010-05-28]
- ANONYM: "LEONTOPODIUM ALPINUM STEMS G", ISTITUTO DI RICERCHE BIOTECNOLOGICHE - BROCHURE, 8. März 2010 (2010-03-08), Seiten 1-4, XP007912102,
- "Edelweiss - ein neuer Stern in der Kosmetikindustrie", SCHWEIZERISCHE LABORATORIUMS ZEITSCHRIFT, SCHWEIZERISCHE LABORPERSONAL VERBAND, BASEL, CH, Bd. 64, Nr. 5, 1. Januar 2007 (2007-01-01) , Seiten 214-215, XP009130606, ISSN: 0253-5211

## Beschreibung

Die vorliegende Erfindung betrifft die kosmetische Verwendung eines Mittels, enthaltend einen Extrakt aus Leontopodium zur Förderung des Haarwachstums und zur Kräftigung der Haarstruktur.

Die menschliche Haut mit ihren Anhangsgebilden ist ein sehr komplex aufgebautes Organ, welches aus einer Vielzahl verschiedener Zelltypen besteht. Jede lebende Zelle dieses Organs ist in der Lage auf Signale ihrer inneren und äußeren Umwelt, zu reagieren. Diese Reaktionen der Zellen werden durch eine geordnete Regulation auf Gen- und Proteinebene realisiert, so dass der Metabolismus von Zellen der Haut und Ihrer Anhangsgebilde nicht statisch sondern sehr dynamisch ist. Die Reaktionen der Haut und/oder ihrer Anhangsgebilde auf Veränderungen der Umgebung dürfen jedoch nicht als Reaktionen einzelner, isolierter Zellen betrachtet werden. Vielmehr ist jede Zelle in ein komplexes Kommunikationsnetzwerk eingebunden. Dieses Netzwerk beinhaltet z.B. die Kommunikation zwischen Zellen der Epidermis und Zellen der Dermis. An der Kommunikation zwischen den Zellen der Haut und/oder ihrer Anhangsgebilde sind Signalmoleküle wie z.B. Interleukine, Wachstumsfaktoren (z.B. KGF, EGF oder FGF) usw. beteiligt.

Der Alterungsprozess ist ein grundlegender biologischer Prozess, der bei nahezu allen lebenden Organismen zu finden ist. Dementsprechend ist auch die menschliche Haut von diesem Phänomen betroffen. Die Hautalterung stellt sich als progressiver Vorgang dar, der zu einem Verlust der Hauthomöostase führt. Er wird von endogenen und exogenen Faktoren beeinflusst. Während die endogenen Aspekte als generisch gesteuertes Programm" ablaufen, sind für die exogenen Faktoren Umwelteinflüsse wie beispielsweise UV-Licht verantwortlich.
Die Menge eines haaraktiven Wirkstoffes, der üblicherweise transdermal und speziell transfollikulär bis zum Haarbulbus penetrieren kann, ist äußerst gering und hängt im Wesentlichen von den physikochemischen Eigenschaften der Substanz selber (z.B: Größe, Ladung, Lipophilie) sowie der Wahl der Formulierung ab.

Haarfollikelzellen unterliegen einem genetisch festgelegten Zyklus von Wachstum, Regression, und Ruhephase. Der Haarfollikel ist damit das einzige Organ, das sich ständig selbst erneuert und somit einen, in Abhängigkeit von der jeweiligen Wachstumsphase, einzigartigen Metabolismus aufweist. So kommt der Metabolismus des Haarfollikels in der Ruhephase fast völlig zum Erliegen und wird mit jedem neuem Beginn eines weiteren Zyklus ebenfalls neu initiiert. Gesteuert wird dieser Zyklus von einer kleinen, hochspezialisierten Zellpopulation im Haarbulbus, den dermalen Papillenzellen, die durch ein komplexes Set molekularer Signale, das spezifisch für jede Phase des Haarzyklus ist, das Haarwachstum kontrollieren.

Extrakte aus Leontopodium werden bereits seit geraumer Zeit in kosmetischen Mitteln eingesetzt.
So beschreibt die internationale Patentanmeldung WO 2001/47538 A1 beispielsweise den Einsatz von Leontopodium Extrakten als UV-Schutzmittel in Kosmetika.

Es wurde nun überraschenderweise gefunden, dass bei der Applikation von Mitteln enthaltend Wirkstoffe oder Wirkstoffgemische, erhältlich aus Pflanzen der Gattung Leontopodium, die Haarwurzel reaktiviert und das Haarwachstum signifikant verbessert wird. Ferner wurde überraschenderweise gefunden, dass diese Mittel geeignet sind das Haar zu kräftigen und / oder zu verdicken. Die vorliegende Erfindung betrifft daher die Verwendung eines Wirkstoffs oder Wirkstoffgemischs, erhältlich aus Pflanzen der Gattung Leontopodium, zur Vitalisierung von Haaren.

Die Steigerung der Haarvitalität durch die erfindungsgemäße Verwendung der Wirkstoffe und Wirkstoffgemische aus Pflanzen der Gattung Leontopodium ist beispielhaft aus den folgenden Beobachtungen ableitbar:
- der Stimulierung der Ausschüttung von Wachstumsfaktoren
- der Stimulierung der Zellproliferation
- der Stimulierung der Haarkeratin-Expression, insbesondere der Expression der Haarkeratine KRT33A und KRT34
- der Verbesserung des Griffs der Haare
- der Verbesserung der Kämmbarkeit der Haare
- der Verbesserung des Glanzes der Haare
- der Erhöhung der Haarfestigkeit
- der Erhöhung der Haardicke
- der Verbesserung des Haarwachstums

Die Verwendung der Wirkstoffe und Wirkstoffgemische aus Pflanzen der Gattung Leontopodium ermöglicht die Entwicklung neuer Produkt- und Wirkkonzepte für biologisch aktive Haarpflegeprodukte, die dem Absinken der Zellvitalität, insbesondere dem Nachlassen der Keratinsynthese und der Abnahme der Zellteilungsakfivität entgegenwirken oder sie rückgängig machen. Die erfindungsgemäße Verwendung von Wirkstoffen und Wirkstoffgemischen aus Pflanzen der Gattung Leontopodium in kosmetischen Haarbehandlungsmitteln wirkt weiterhin der gesteigerten Mikroinflammation und Apoptose entgegen oder macht sie rückgängig.

### Zusammenfassend sind die

- Verwendung eines Wirkstoffs oder Wirkstoffgemischs, erhältlich aus Pflanzen der Gattung Leontopodium, zur Erhöhung der Haarfestigkeit;
- Verwendung eines Wirkstoffs oder Wirkstoffgemischs, erhältlich aus Pflanzen der Gattung Leontopodium, zur Verbesserung der Haarstruktur;
- Verwendung eines Wirkstoffs oder Wirkstoffgemischs, erhältlich aus Pflanzen der Gattung Leontopodium, zur Verbesserung des Haarwachstums;
- Verwendung eines Wirkstoffs oder Wirkstoffgemischs, erhältlich aus Pflanzen der Gattung Leontopodium, zur Stimulierung der Synthese der von der Alterung betroffenen Haarkeratine KRT33A und KRT34;
- Verwendung eines Wirkstoffs oder Wirkstoffgemischs, erhältlich aus Pflanzen der Gattung Leontopodium, zur Förderung der Keratinsynthese im Haar;
- Verwendung eines Wirkstoffs oder Wirkstoffgemischs, erhältlich aus Pflanzen der Gattung Leontopodium, zur Stimulation der Zellvitalität, der Zellproliferation sowie der Ausschüttung von Wachstumsfaktoren stimuliert und zur Inhibierung der Aktivierung von katagenassoziierten Parametern;
- Verwendung eines Wirkstoffs oder Wirkstoffgemischs, erhältlich aus Pflanzen der Gattung Leontopodium, zur Verbesserung der Kämmbarkeit der keratinischen Fasern;
weitere bevorzugte Gegenstände der vorliegenden Anmeldung.

Erfindungsgemäß bevorzugt verwendete Mittel enthalten den Wirkstoff oder das Wirkstoffgemisch, erhältlich aus Pflanzen der Gattung Leontopodium in Mengen von 0,0001 bis 5,0 Gew.-%, vorzugsweise 0,001 bis 4,0 Gew.-% und insbesondere 0,01 bis 3,0 Gew.-%, jeweils bezogen auf das Gesamtgewicht des Mittels.

Die erfindungsgemäß verwendeten Wirkstoffe oder Wirkstoffgemische werden vorzugsweise aus Pflanzenzubereitungen, insbesondere Presssäften und / oder Extrakten der Pflanzen der Arten Leontopodium alpinum, Leontopodium stracheyi, Leontopodium nivale, Leontopodium palibinianum, Leontopodium souliei, Leontopodium lineariofolium oder Leontopodium gnaphalioides, bevorzugt aus Presssäften und / oder Extrakten der Pflanzen der Arten Leontopodium alpinum, Leontopodium stracheyi und insbesondere bevorzugt aus Presssäften und / oder Extrakten der Pflanzen der Art Leontopodium alpinum gewonnen.

Als Wirkstoff sind bevorzugt solche Aktivsubstanzen zu verstehen, die in mindestens einer Pflanzenart der Gattung Leontopodium, aber nicht ubiquitär im Pflanzenreich vorkommen, d.h. solche Stoffe, die in jeder oder fast jeder Pflanze (irgendeiner Gattung) zu finden sind; insbesondere Wasser, Einfachzucker, Chlorophyll etc. zählen nicht zu den erfindungsgemäßen Wirkstoffen. Allerdings können solche ubiquitär in Pflanzen vorkommenden Stoffe zusätzlich zu den Aktivstoffen/Wirkstoffen in den Wirkstoffgemischen, erhältlich aus Pflanzen der Gattung Leontopodium, enthalten sein. Gemäß einer anderen bevorzugten Ausführungsform ist der Wirkstoff ausgewählt ausgewählt aus der Gruppe der Flavonoide und der Gerbstoffe.

Ein Wirkstoffgemisch enthält bevorzugt mindestens eine, bevorzugt mindestens zwei oder mehrere Aktivsubstanzen, die in mindestens einer Pflanzenart der Gattung Leontopodium vorkommen. Gemäß einer besonderen Ausführungsform ist das Wirkstoffgemisch ausgewählt aus Pflanzenzubereitungen, insbesondere Presssäften und Extrakten, die aus Pflanzen der Gattung Leontopodium, vorzugsweise aus Pflanzen der Art Leontopodium alpinum gewonnen werden.

Unter einer Pflanzenzubereitung, erhältlich aus Pflanzen der Gattung Leontopodium, sind erfindungsgemäß die Pflanze selbst, ihre Pflanzenteile, Extrakte und Presssäfte aus Leontopodium alpinum, Leontopodium stracheyi, Leontopodium nivale, Leontopodium palibinianum, Leontopodium souliei, Leontopodium lineariofolium oder Leontopodium gnaphalioides zu verstehen.

Gemäß einer besonderen Ausführungsform werden der Wirkstoffe, die Wirkstoffgemische, insbesondere die Pflanzenzubereitungen, insbesondere bevorzugt die Presssäfte und/oder Extrakte, aus Pflanzen der Art Leontopodium alpinum gewonnen.

Bevorzugt werden die Presssäfte bzw. Extrakte aus Kraut (den oberirdischen Pflanzenteilen) und/oder Wurzel gewonnen. Bevorzugter Weise werden die Presssäfte mit mechanischer Pressung gewonnen.

Ein Extrakt im Sinne der vorliegenden Anmeldung ist ein Stoff oder Stoffgemisch, welches durch Extraktion und teilweises oder völliges Eindampfen der Extraktionslösung gewonnen wurde. Man unterscheidet nach der Beschaffenheit *Trockenextrakte* d.h. bis zur Trockene eingedampfte Extrakte, *Fluidextrakte* d.h. mit Lösungsmitteln so hergestellte Extrakte, dass aus einem Teil Droge höchstens 2 Teile Fluid-Extrakt gewonnen werden, *zähflüssige Extrakte bzw. Dickextrakte,* d.h. Extrakte, bei denen ein Teil des Lösungsmittels verdampft wird.

Die in den erfindungsgemäß verwendeten Extrakte bzw. Presssäfte können aus den erntefrischen Pflanzen, aber auch aus abgelagerter Ware erhalten werden.

Die Extrakte können mit Wasser, sowie polaren oder unpolaren organischen Lösungsmitteln sowie Mischungen davon in dem Fachmann bekannter Weise hergestellt werden. Die erfindungsgemäß verwendeten Extrakte werden durch Extraktion vorzugsweise mit organischen Lösemitteln, Wasser oder Gemischen daraus, gewonnen. Bevorzugt geeignete organische Lösemittel sind Ketone (z.B. Aceton), Ether, Ester, Alkohole oder halogenierte Kohlenwasserstoffe. Besonders bevorzugte Extraktionsmittel sind Wasser und/oder Alkohole. Unter den Alkoholen sind dabei (C₁ bis C₆)- Alkohole, wie Ethanol und Isopropanol und zwar sowohl als alleiniges Extraktionsmittel als auch in Mischung mit Wasser, bevorzugt. Besonders bevorzugte Extraktionsmittel sind Wasser, Ethanol, 2-Propanol, 1,2-Propylenglykol, 1,3-Butylenglykol, ganz besonders bevorzugt sind Wasser, Ethanol, 2-Propanol und 1,2-Propylenglykol sowie Mischungen hiervon, z. B. eine Mischung 1,2-Propylenglykol/Wasser im Verhältnis 4:1. Extrakte, die durch Extraktion mit Ethanol oder Wasser/Ethanol-Mischungen, erhalten werden können, sowie Presssaft, sind besonders bevorzugt.
Es können sowohl die Extrakte sowohl im ursprünglichen Extraktionsmittel als auch Extrakte/Presssaft in Wasser oder anderen organischen Lösungsmitteln und/oder deren Gemisch, insbesondere Ethanol sowie Ethanol/Wasser-Mischungen eingesetzt werden. Bevorzugt wird extrahiertes oder gepresstes Material als Feststoff eingesetzt, dem das Lösungsmittel (insbesondere möglichst schonend) entzogen wurde. Es können aber auch solche Extrakte/Presssäfte eingesetzt werden, denen das Lösungsmittel zum Teil entzogen wurde, so dass ein verdickter Extrakt/Presssaft eingesetzt wird. Ganz besonders bevorzugt sind Presssäfte aus dem frischen Leontopodium alpinum. Insbesondere werden die Extrakte und/oder Presssäfte in fester Form eingesetzt. Die Extraktion wird bevorzugt bei einer Temperatur von 25°C bis 90°C durchgeführt.

Es kann in einer anderen Ausführungsform auch bevorzugt sein, dass das erfindungsgemäß zu verwendende Wirkstoffgemisch, bevorzugt die Pflanzenzubereitungen dadurch gekennzeichnet sind, dass der Extrakt bzw. der Presssaft mindestens ein polares Lösungsmittel, ausgewählt aus Ethanol, 1-Propanol, 2-Propanol, 1-Butanol, 2-Butanol, Ethylenglykol, 1,2-Propylenglykol, 1,3-Butylenglykol, Glycerin und Wasser, sowie Mischungen hiervon enthält.

Je nach Wahl der Extraktionsmittel kann es bevorzugt sein, den Extrakt durch Zugabe eines Lösungsvermittlers zu stabilisieren. Als Lösungsvermittler geeignet sind z. B. Ethoxylierungsprodukte von gegebenenfalls gehärteten pflanzlichen und tierischen Ölen. Bevorzugte Lösungsvermittler sind ethoxylierte Mono-, Di- und Triglyceride von C8-22-Fettsäuren mit 4 bis 50 Ethylenoxid-Einheiten, z. B. hydriertes ethoxyliertes Castoröl, Olivenölethoxylat, Mandelölethoxylat, Nerzölethoxylat, Polyoxyethylenglykolcapryl-/caprinsäureglyceride, Polyoxyethylenglycerinmonolaurat und Polyoxyethylenglykolkokos-fettsäureglyceride. Besonders bevorzugt ist Olivenölethoxylat (INCI-Bezeichnung: PEG-10 Olive Glycerides).

Die Trockenmasse des Extraktes bzw. des Presssaftes ist abhängig von der Molmasse und der Löslichkeit der dispergierten Inhaltsstoffe und beträgt in der Regel, jeweils bezogen auf das Gewicht des Extraktes bzw. des Presssaftes, 1 bis 80 Gew.-%. Bevorzugt beträgt die Trockenmasse 15 bis 50 Gew.-% und besonders bevorzugt 20 bis 35 Gew.-%. Bei einem Molekulargewicht der Inhaltsstoffe über 100.000 Dalton kann eine Trockenmasse von 1 bis 20 Gew.-% bevorzugt und eine Trockenmasse von 1 bis 10 Gew.-% besonders bevorzugt sein. Besonders bevorzugte kosmetische Mittel sind dadurch gekennzeichnet, dass die Trockenmasse des Extraktes bzw. des Presssaftes 5 - 80 Gew.-%, bezogen auf das Gewicht des Extraktes bzw. des Presssaftes, beträgt.

Die erfindungsgemäße Verwendung ist vorzugsweise kosmetisch und nicht therapeutisch. Die Verwendung erfolgt zudem vorzugsweise topisch, also beispielsweise durch Auftragen oder Aufsprühen auf das Haar oder die Haut. Der Wirkstoffs oder das Wirkstoffgemisch, erhältlich aus Pflanzen der Gattung Leontopodium, liegt daher vorzugsweise in einem kosmetischen Träger bzw. als Bestandteil eines kosmetischen Mittels vor. Ein weiterer Gegenstand der vorliegenden Erfindung ist die Verwendung eines Wirkstoffs oder Wirkstoffgemischs, erhältlich aus Pflanzen der Gattung Leontopodium zur Herstellung kosmetischer Mittel zur Vitalisierung von Haaren, insbesondere für
- die Stimulierung der Ausschüttung von Wachstumsfaktoren
- die Stimulierung der Zellproliferation
- die Stimulierung der Haarkeratin-Expression, insbesondere der Expression der Haarkeratine KRT33A und KRT34
- die Förderung der Keratinsynthese im Haar
- die Verbesserung des Griffs der Haare
- die Verbesserung der Kämmbarkeit der Haare
- die Verbesserung des Glanzes der Haare
- die Erhöhung der Haarfestigkeit
- die Erhöhung der Haardicke
- die Verbesserung des Haarwachstums

Erfindungsgemäß bevorzugt verwendete kosmetische Mittel enthalten den Wirkstoff oder das Wirkstoffgemisch, erhältlich aus Pflanzen der Gattung Leontopodium in Mengen von 0,0001 bis 5,0 Gew.-%, vorzugsweise 0,001 bis 4,0 Gew.-% und insbesondere 0,01 bis 3,0 Gew.-%, jeweils bezogen auf das Gesamtgewicht des kosmetischen Mittels.

Hinsichtlich der Art der kosmetischen Mittel, in denen der Wirkstoff oder das Wirkstoffgemisch aus Pflanzen der Gattung Leontopodium verwendet wird, bestehen keine prinzipiellen Einschränkungen. Als Konfektionierung dieser Mittel sind beispielsweise Cremes, Lotionen, Lösungen, Wässer, Emulsionen wie W/O-, O/W-, PIT-Emulsionen (Emulsionen nach der Lehre der Phaseninversion, PIT genannt), Mikroemulsionen und multiple Emulsionen, grobe, instabile, ein oder mehrphasige Schüttelmixturen, Gele, Sprays, Aerosole und Schaumaerosole geeignet. Erfindungsgemäß bevorzugte Verwendungen sind daher dadurch gekennzeichnet, dass das kosmetische Mittel ein Haarbehandlungsmittel, insbesondere Shampoo, Haamachspülmittel, Haargel, Haarwasser, Haarkur, Haarcreme, Haarlotion, Haarspray und Haartinktur ist.

Die Verwendung eines Wirkstoffs oder Wirkstoffgemischs, erhältlich aus Pflanzen der Gattung Leontopodium in auf der Haut und dem Haar verbleibenden Zubereitungen hat sich als besonders wirksam erwiesen und kann daher bevorzugte Ausführungsformen der erfindungsgemäßen Lehre darstellen. Unter auf der Haut und dem Haar verbleibend werden erfindungsgemäß solche Zubereitungen verstanden, die nicht im Rahmen der Behandlung nach einem Zeitraum von wenigen Sekunden bis zu einer Stunde mit Hilfe von Wasser oder einer wässrigen Lösung wieder von der Haut ab- oder aus dem Haar ausgespült werden. Vielmehr verbleiben die Zubereitungen bis zur nächsten Wäsche auf der Haut oder dem Haar.

Gemäß einer bevorzugten Ausführungsform der Erfindung wird der Wirkstoff oder das Wirkstoffgemischs, erhältlich aus Pflanzen der Gattung Leontopodium, in Haarkuren oder Haarkonditionern verwendet. Diese Zubereitungen können nach Ablauf einer Einwirkzeit mit Wasser oder einem zumindest überwiegend wasserhaltigen Mittel ausgespült werden; bevorzugt werden sie jedoch, wie oben ausgeführt, auf dem Haar belassen.

In einer besonders bevorzugten Ausführungsform wird der aus Pflanzen der Gattung Leontopodium erhältliche Wirkstoff bzw. das aus Pflanzen der Gattung Leontopodium erhältlich Wirkstoffgemisch in Kombination mit mindestens einem weiteren Haarwuchs stimulierenden Wirkstoff eingesetzt. Diese, den Haarausfall verhindernden, lindernden oder heilenden, insbesondere die Haarwurzel stabilisierenden Wirkstoffe werden nachstehend beschrieben:
Propecia (Finasterid) ist das zur Zeit einzige Präparat, das weltweit zugelassen ist und für das in zahlreichen Studien eine Wirksamkeit und Verträglichkeit nachgewiesen wurde. Propecia bewirkt, dass sich weniger DHT aus Testosteron bilden kann.
Minoxidil ist mit oder ohne ergänzende Zusatzstoffe das wohl älteste nachweislich wirkende Haarwuchsmittel. Zur Behandlung von Haarausfall darf es nur zur äußeren Anwendung verwendet werden. Es gibt Haarwasser, die 2%-5% Minoxidil enthalten, außerdem Gels mit bis zu 15% Minoxidil. Die Wirksamkeit nimmt mit der Dosierung zu, in Haarwassern ist Minoxidil jedoch nur bis zu 5% Anteil löslich. In vielen Ländern sind Haarwasser mit bis zu 2% Minoxidilgehalt verschreibungsfrei erhältliche. Zur Bekämpfung der hormonellen Einflüsse auf die Haarfollikel kann zur äußeren Anwendung Spironolactone in Form von Haarwasser und in Kombination mit Minoxidil angewandt werden. Spironolactone wirkt als Androgen-Rezeptor-Blocker, d.h. die Bindung von DHT an die Haarfollikel wird verhindert.
Zusammenfassend ist eine erfindungsgemäße Verwendung bevorzugt, bei welcher ein kosmetisches Mittel eingesetzt wird, das neben einem Wirkstoff oder Wirkstoffgemisch, erhältlich aus Pflanzen der Gattung Leontopodium, bezogen auf sein Gesamtgewicht 0,001 bis 5 Gew.-% Haarwurzelstabilisierende Stoffe, insbesondere Minoxidil und/oder Finasterid und/oder Ketoconazol, enthält.

Durch zusätzliche Antischuppenwirkstoffe (beispielsweise Climbazol, Piroctone Olamine oder Zink-Pyrithion) wird die Menge des Schuppen verursachenden Hefepilzes gezielt reduziert, die Keimflora erreicht wieder die normale prozentuale Zusammensetzung und die Abschuppung wird auf das physiologische Maß reduziert. Labortests haben jedoch nachgewiesen, dass die unterschiedlichen Artvertreter des Pityrosporum ovale unterschiedlich gut auf die Antischuppenwirkstoffe reagieren. Um alle Schuppenerreger maximal zu bekämpfen ist daher eine Kombination von Anti-Schuppenwirkstoffen am erfolgreichsten.

Zusammenfassend ist eine erfindungsgemäße Verwendung bevorzugt, bei welcher ein kosmetisches Mittel eingesetzt wird, das neben einem Wirkstoff oder Wirkstoffgemisch, erhältlich aus Pflanzen der Gattung Leontopodium, bezogen auf sein Gesamtgewicht 0,001 bis 5 Gew.-% Antischuppenwirkstoffe, insbesondere Piroctone Olamine (1-Hydroxy-4-methyl-6-(2,4,4-trirnethylpentyl)pyridin-2(1H)-on, Verbindung mit 2-Aminoethanol, 1:1) und/oder Zink-Pyrithion und/oder Selensulfid und/oder Climbazol und/oder Salicylsäure oder Fumarsäure, enthält.

In einer weiteren besonders bevorzugten Ausführungsform wird der aus Pflanzen der Gattung Leontopodium erhältliche Wirkstoff bzw. das aus Pflanzen der Gattung Leontopodium erhältlich Wirkstoffgemisch in Kombination mit mindestens einem das Haar pflegenden Wirkstoff eingesetzt. Diese Wirkstoffe werden nachstehend beschrieben:
Eine bevorzugte Gruppe von haarpflegenden Inhaltsstoffen der erfindungsgemäß verwendeten Mittel sind Vitamine, Provitamine oder Vitaminvorstufen. Diese werden nachfolgend beschrieben:
   Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäß verwendeten Mittel enthalten die Vitamin A-Komponente bevorzugt in Mengen von 0,05-1 Gew.-%, bezogen auf die gesamte Zubereitung.
Zur Vitamin B-Gruppe oder zu dem Vitamin B-Komplex gehören u. a. Vitamin B₁ (Thiamin), Vitamin B₂ (Riboflavin), Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist. Ebenfalls dazu gehört Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat. Die genannten Verbindungen des Vitamin B₅-Typs sind in den erfindungsgemäß verwendeten Mitteln bevorzugt in Mengen von 0,05 - 10 Gew.-%, bezogen auf das gesamte Mittel, enthalten. Mengen von 0,1 - 5 Gew.-% sind besonders bevorzugt. Weiterhin kann Vitamin B₆ (Pyridoxin sowie Pyridoxamin und Pyridoxal) eingesetzt werden. Vitamin C (Ascorbinsäure). Vitamin C wird in den erfindungsgemäß verwendeten Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein. Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäß verwendeten Mitteln bevorzugt in Mengen von 0,05-1 Gew.-%, bezogen auf das gesamte Mittel, enthalten. Vitamin F. Unter dem Begriff "Vitamin F" werden üblicherweise essentielle Fettsäuren, insbesondere Linolsäure, Linolensäure und Arachidonsäure, verstanden. Vitamin H. Als Vitamin H wird die Verbindung (3a*S*,4*S*, 6a*R*)-2-Oxohexahydrothienol[3,4-*d*]-imidazol-4-valeriansäure bezeichnet, für die sich aber inzwischen der Trivialname Biotin durchgesetzt hat. Biotin ist in den erfindungsgemäß verwendeten Mitteln bevorzugt in Mengen von 0,0001 bis 1,0 Ge Besonders bevorzugt sind Kombinationen von Pressäften, gewonnen aus Leontopodium, mit Tocopherolen, insbesondere alpha-Tocopherot, sowie Kombinationen von Extrakten aus Leontopodium mit Tocopherolen, insbesondere alpha-Tocopherol.w.%, insbesondere in Mengen von 0,001 bis 0,01 Gew.-% enthalten.

Besonders bevorzugte erfindungsgemäß verwendete Mittel sind dadurch gekennzeichnet, dass sie als Pflegestoff - bezogen auf ihr Gewicht - 0,0001 bis 1 Gew.-%, bevorzugt 0,001 bis 0,5 Gew.-% und besonders bevorzugt 0,005 bis 0,1 Gew.-% mindestens eines Ubichinons und/oder mindestens eines Ubichinols und/oder mindestens eines Derivates dieser Substanzen enthalten, wobei besonders bevorzugt zu verwendende Mittel Coenzym Q 10, bevorzugt zu 0,005 bis 0,1 Gew.-% enthalten.

Alternativ zu den besonders bevorzugten Ubichinonen oder zusätzlich zu ihnen können die erfindungsgemäß verwendeten Mittel auch Plastochinone (polyprenylierte 2,3-Dimethylbenzochinon-Derivate) enthalten. Hier sind bevorzugte erfindungsgemäß verwendete Mittel dadurch gekennzeichnet, dass sie 0,0002 bis 4 Gew.-%, vorzugsweise 0,0005 bis 3 Gew.-%, besonders bevorzugt 0,001 bis 2 Gew.-%, weiter bevorzugt 0,0015 bis 1 und insbesondere 0,002 bis 0,5 Gew.-% mindestens eines Plastochinon enthalten. Dabei enthält die Prenylseitenkette n Prenyleinheiten. Bevorzugt sind Werte für n von 1 bis 20, vorzugsweise von 2 bis 15 und insbesondere für 5, 6, 7, 8, 9, 10 steht, wobei besonders bevorzugt zu verwendende Mittel ein Plastochinon mit n=9 enthalten. Besonders bevorzugt ist eine Kombination von Presssäften oder Extrakten aus Leontopodium mit Coenzym Q10.

Als weiteren Inhaltsstoff können die erfindungsgemäß verwendeten kosmetischen Mittel mindestens ein Peptid oder ein Protein oder ein Proteinhydrolysat, wobei Proteinhydrolysate bevorzugt sind. Proteinhydrolysate sind Produktgemische, die durch sauer, basisch oder enzymatisch katalysierten Abbau von Proteinen (Eiweißen) erhalten werden. Erfindungsgemäß können Proteinhydrolysate sowohl pflanzlichen als auch tierischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Erfindungsgemäß bevorzugt ist die Verwendung von Proteinhydrolysaten pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex) und Crotein^{®} (Croda) erhältlich.

Vorzugsweise wird unabhängig von der Wahl des X in Formel (P-I) der Rest R" aus Keratin oder keratinhydrolxysaten ausgewählt. Bevorzugt verwendete kosmetische Mittel sind dadurch gekennzeichnet, dass sie mindestens ein Proteolipid der Formel (P-I) enthalten, in der R" für Keratin oder ein Keratinhydrolysat steht.

Insbesondere ist die Verwendung kosmetischer Mittel bevorzugt, die mindestens ein Proteolipd der Formel (P-I) enthalten, in der R^{III} -CH₃ bedeutet und R^{IV} für -(CH₂)ₓ- mit x = 0, 1, 2, 3, 4, 5, 6, 7, 8 steht.

Weiter sind besonders bevorzugte Verwendung Mittel dadurch gekennzeichnet, dass die kosmetischen Mittel mindestens ein Proteolipd der Formel (I) enthalten, in der X für -N⁺(CH₃)₂-CH₂-CH(OH)-CH₂-und R' für -(CH₂)₁₇-CH₃ steht.

Ebenfalls weiter bevorzugt verwendete erfindungsgemäße kosmetische Mittel dadurch gekennzeichnet, dass sie mindestens ein Proteolipd der Formel (P-I) enthalten, in der X für -C(O)-O-und R' für -(CH₂)₁₇-CH₃ steht.

Es hat sich als vorteilhaft erwiesen, zusätzlich zu den Proteolipiden Proteinhydrolysate einzusetzen. Diese verstärken die Wirkung der Proteolipide und werden ihrerseits in ihren Effekten verstärkt. Die Proteinhydrolysate wurden weiter oben als Rest R" detailliert beschrieben. Zusammenfassend kosmetische Mittel bevorzugt verwendet, die zusätzlich - bezogen auf ihr Gewicht - 0,01 bis 10 Gew.-%, vorzugsweise 0,05 bis 7 Gew.-%, besonders bevorzugt 0,1 bis 5 Gew.-%, weiter bevorzugt 0,25 bis 2,5 Gew.-% und insbesondere 0,5 bis 2,0 Gew.-% Proteinhydrolysat(e), vorzugsweise Keratinhydrolysat(e) enthalten.

Als weiteren Inhaltsstoff können die erfindungsgemäß verwendeten Mittel mit besonderem Vorzug eine oder mehrere Aminosäuren enthalten. Erfindungsgemäß besonders bevorzugt einsetzbare Aminosäuren stammen aus der Gruppe Glycin, Alanin, Valin, Leucin, Isoleucin, Phenylalanin, Tyrosin, Tryptophan, Prolin, Asparaginsäure, Glutaminsäure, Asparagin, Glutamin, Serin, Threonin, Cystein, Methionin, Lysin, Arginin, Histidin, (3-Alanin, 4-Aminobuttersäure (GABA), Betain, L-Cystin (L-Cyss), L-Citrullin, L-Theanin, 3 ',4 '-Dihydroxy-L-phenylalanin (L-Dopa), 5 '-Hydroxy-L-tryptophan, L-Homocystein, S-Methyl-L-methionin, S-Allyl-L-cystein-sulfoxid (L-Allün), L-trans-4-Hydroxyprolin, L-5-Oxoprolin (L-Pyroglutaminsäure), L-Phosphoserin, Kreatin, 3-Methyl-L-histidin, L-Omithin, wobei sowohl die einzelnen Aminosäuren als auch Mischungen eingesetzt werden können. Bevorzugte erfindungsgemäß verwendete Mittel enthalten eine oder mehrere Aminosäuren in engeren Mengenbereichen. Hier sind erfindungsgemäß bevorzugte Haarbehandlungsmittel dadurch gekennzeichnet, daß sie als Pflegestoff - bezogen auf ihr Gewicht - 0,01 bis 5 Gew.-%, vorzugsweise 0,02 bis 2,5 Gew.-%, besonders bevorzugt 0,05 bis 1,5 Gew.-%, weiter bevorzugt 0,075 bis 1 Gew.% und insbesondere 0,1 bis 0,25 Gew.-% Aminosäure(n), vorzugsweise aus der Gruppe Glycin und/oder Alanin und/oder Valin und/oder Lysin und/oder Leucin und/oder Threonin enthalten.

Besonders bevorzugt ist eine Kombination von einem Pressaft aus Leontopodium mit Glycin, einem Pressaft aus Leontopodium mit Alanin, einem Pressaft aus Leontopodium mit Valin, einem Pressaft aus Leontopodium mit Lysin, einem Pressaft aus Leontopodium mit Leucin, einem Pressaft aus Leontopodium mit Threonin. Weiterhin besonders bevorzugt sind Kombinationen von einem Extrakt aus Leontopodium mit Glycin, einem Extrakt aus Leontopodium mit Alanin, einem Extrakt aus Leontopodium mit Valin, einem Extrakt aus Leontopodium mit Lysin, einem Extrakt aus Leontopodium mit Leucin, einem Extrakt aus Leontopodium mit Threonin.

Erfindungsgemäß bevorzugt zu verwendende Mittel enthalten als Pflegestoff - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% mindestens eines 2-Furanonderivats der Formel (Fur-I) und/oder der Formel (Fur-II)

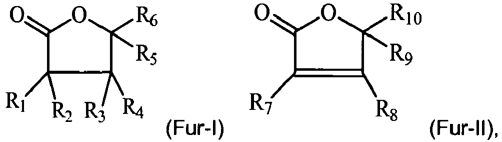

Für erfindungsgemäß zu verwendende furanonderviathaltige Mittel wird Schutz begehrt wird oder kann Schutz begehrt werden; 2-Furanonderivate tragen zum technischen Ziel der Erfindung und somit zur Lösung der der anmeldungsgemäßen Erfindung zugrunde liegenden technischen Aufgabe bei.

Ein weiterer, bevorzugter einsetzbarer Pflegestoff, der aktivierende Eigenschaften besitzt, ist das Taurin. Erfindungsgemäß bevorzugt zu verwendende Mittel enthalten als Pflegestoff - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% Taurin (2-Aminoethansulfonsäure).

Eine weitere Gruppe, bevorzugter einsetzbarer Pflegestoff, die aktivierende Eigenschaften besitzen sind die Purine und Purinderivate. Erfindungsgemäß bevorzugt zu verwendende Mittel enthalten als Pflegestoff - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.% Purin und/oder Purinderivate, insbesondere Coffein.

Schließlich können die erfindungsgemäß verwendeten Mittel auch weitere Pflanzenextrakte enthalten. Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.
Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdom, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.
Besonders bevorzugt sind die Extrakte aus Grünem Tee, Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel.
Ganz besonders für die erfindungsgemäße Verwendung geeignet sind die Extrakte aus Grünem Tee, Mandel, Aloe Vera, Kokosnuß, Mango, Aprikose, Limone, Weizen, Kiwi und Melone.
Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.
Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.
Weiterhin kann es bevorzugt sein, in den erfindungsgemäß verwendeten Mitteln Mischungen aus mehreren, insbesondere aus zwei, verschiedenen Pflanzenextrakten einzusetzen.

Gemäß einer bevorzugten Ausführungsform ist zusätzlich bevorzugt mindestens ein Haarkonditioniermittel, ausgewählt aus kationischen Polymeren, kationischen Tensiden, Silikonen und/oder pflanzlichen Ölen, enthalten.

Die erfindungsgemäß zu verwendenden Zusammensetzungen können Tenside, insbesondere kationische Tenside, enthalten. Für erfindungsgemäß zu verwendende tensidhaltige Mittel wird Schutz begehrt wird oder kann Schutz begehrt werden; Tenside, insbesondere kationische Tenside, tragen zum technischen Ziel der Erfindung und somit zur Lösung der der anmeldungsgemäßen Erfindung zugrunde liegenden technischen Aufgabe bei.

Besonders bevorzugt verwendete kosmetische Mittel sind dadurch gekennzeichnet, dass sie als kationischen Pflegestoff - bezogen auf ihr Gewicht - 0,05 bis 7,5 Gew.-%, vorzugsweise 0,1 bis 5 Gew.-%, besonders bevorzugt 0,2 bis 3,5 Gew.-% und insbesondere 0,25 bis 2,5 Gew.-% kationische(s) Tensid(e) aus der Gruppe der quartären Ammoniumverbindungen und/oder der Esterquats und/oder der Amidoamine enthalten, wobei bevorzugte kationische(s) Tensid(e) ausgewählt ist/sind aus Alkyltrimethylammoniumchloriden mit vorzugsweise 10 bis 18 Kohlenstoffatomen im Alkylrest und/oder Dialkyldimethylammoniumchloride mit vorzugsweise 10 bis 18 Kohlenstoffatomen im Alkylrest und/oder Trialkylmethylammoniumchloride mit vorzugsweise 10 bis 18 Kohlenstoffatomen im Alkylrest und/oder Cetyltrimethylammoniumchlorid und/oder Stearyltrimethylammoniumchlorid und/oder Distearyldimethylammoniumchlorid und/oder Lauryldimethylammoniumchlorid und/oder Lauryldimethylbenzylammoniumchlorid und/oder Tricetylmethylammoniumchlorid und/oder Quatemium-27 und/oder Quaternium-83 und/oder N-Methy)-N(2-hydroxyethy))-N,N-(ditalgacyloxyethyl)ammonium-methosulfat und/oder N-Methyl-N(2-hydroxyethyl)-N,N-(distearoyloxyethyl)ammonium-methosulfat und/oder N,N-Dimethyl-N,N-distearoyloxyethyl-ammoniumchlorid und/oder N,N-Di-(2-hydroxyethyl)-N,N-(fettsäureesterethyl)-ammoniumchlorid.

Als weiteren optionalen Bestandteil können die erfindungsgemäß verwendeten Mittel 0,01 bis 10 Gew.-% mindestens eines Polymers aus der Gruppe der kationischen und/oder amphoteren Polymere enthalten. Polymere, insbesondere kationische Polymere, tragen zum technischen Ziel der Erfindung und somit zur Lösung der der anmeldungsgemäßen Erfindung zugrunde liegenden technischen Aufgabe bei.

Eine erfindungsgemäße Verwendung, dadurch gekennzeichnet, dass ein kosmetisches Mittel, umfassend
a) einen Wirkstoff oder ein Wirkstoffgemisch, erhältlich aus Pflanzen der Gattung Leontopodium;
b) mindestens einen weiteren von a) verschiedenen Haar pflegenden oder stimulierenden Wirkstoff eingesetzt wird, ist bevorzugt.

Bevorzugt verwendete kosmetische Mittel enthalten neben den zuvor beschrieben Inhaltsstoffe weitere Aktiv- oder Hilfsstoffe, vorzugsweise aus der Gruppe der Tenside, der Polymere, der Penetrationshilfsmittel und der UV-Schutzmittel.

Bevorzugte kosmetische Zubereitungen enthalten mindestens ein anionisches und/oder mindestens ein amphoteres Tensid. Bevorzugt wird eine erfindungsgemäße Verwendung, bei welcher ein kosmetisches Mittel, umfassend
a) einen Wirkstoff oder ein Wirkstoffgemisch, erhältlich aus Pflanzen der Gattung Leontopodium;
b) mindestens ein anionisches und/oder amphoteres Tensid eingesetzt wird.

Erfindungsgemäß bevorzugte kosmetische Mittel sind dadurch gekennzeichnet, dass sie - bezogen auf ihr Gewicht - 5 bis 40 Gew.-%, vorzugsweise 6 bis 35 Gew.-%, weiter bevorzugt 7 bis 30 Gew.-%, noch weiter bevorzugt 8 bis 25 Gew.-% und insbesondere 10 bis 20 Gew.-% anionische(s) Tensid(e) enthalten.

Als anionische Tenside und Emulgatoren eignen sich für die erfindungsgemäßen Mittel alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glycol- oder Polyglycolether-Gruppen, Ester-, Ether- und Amidgruppen sowie Hydroxylgruppen enthalten sein. Beispiele für geeignete anionische Tenside und Emulgatoren sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Acylglutamate der Formel (I), 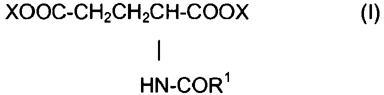 in der R¹CO für einen linearen oder verzweigten Acylrest mit 6 bis 22 Kohlenstoffatomen und 0, 1, 2 oder 3 Doppelbindungen und X für Wasserstoff, ein Alkali- und/oder Erdalkalimetall, Ammonium, Alkylammonium, Alkanolammonium oder Glucammonium steht, beispielsweise Acylglutamate, die sich von Fettsäuren mit 6 bis 22, vorzugsweise 12 bis 18 Kohlenstoffatomen ableiten, wie beispielsweise C_{12/14}- bzw. C_{12/18}-Kokosfettsäure, Laurinsäure, Myristinsäure, Palmitinsäure und/oder Stearinsäure, insbesondere Natrium-N-cocoyl- und Natrium-N-stearoyl-L-glutamat,
- Ester einer hydroxysubstituierten Di- oder Tricarbonsäure der allgemeinen Formel (II), 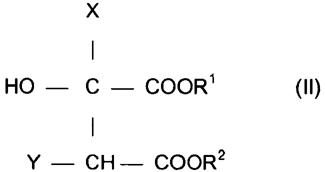 in der X=H oder eine -CH₂COOR-Gruppe ist, Y=H oder -OH ist unter der Bedingung, dass Y=H ist, wenn X=-CH₂COOR ist, R, R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt sind, unter der Maßgabe, dass wenigstens eine der Gruppen R, R¹ oder R² ein Rest Z ist,
- Ester der Sulfobemsteinsäure oder der Sulfosuccinate der allgemeinen Formel (III), 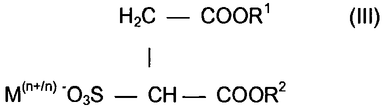 in der M^{(n+/n}) für n = 1 ein Wasserstoffatom, ein Alkalimetallkation, eine Ammoniumgruppe oder das Kation einer ammonium-organischen Base und für n = 2 ein Erdalkalimetallkation darstellt und R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt ist, unter der Maßgabe, dass wenigstens eine der Gruppen R¹ oder R² ein Rest Z ist,
- Sulfobemsteinsäuremono- und -dialkylester mit 8 bis 24 C-Atomen in der Alkylgruppe und Sulfobemsteinsäuremonoalkylpolyoxyethylester mit 8 bis 24 C-Atomen in der Alkylgruppe und 1 bis 6 Oxyethylgruppen,
- Alkylsulfate und Alkylpolyglycolethersulfate der Formel R-(O-CH₂-CH₂)ₓ-OSO₃H, in der R eine bevorzugt lineare Alkylgruppe mit 8 bis 30 C-Atomen und x = 0 oder 1 - 12 ist,
- gemischte oberflächenaktive Hydroxysulfonate gemäß DE-A-37 25 030,
- Ester der Weinsäure und Zitronensäure mit Alkoholen, die Anlagerungsprodukte von etwa 2-15 Molekülen Ethylenoxid und/oder Propylenoxid an C₈₋₂₂-Fettalkohole darstellen,
- Alkyl- und/oder Alkenyletherphosphate,
- sulfatierte Fettsäurealkylenglycolester,
- Monoglyceridsulfate und Monoglyceridethersulfate.

Bevorzugte anionische Tenside und Emulgatoren sind Acylglutamate, Acylisethionate, Acylsarcosinate und Acyltaurate, jeweils mit einem linearen oder verzweigten Acylrest mit 6 bis 22 Kohlenstoffatomen und 0, 1, 2 oder 3 Doppelbindungen, der in besonders bevorzugten Ausführungsformen aus einem Octanoyl-, Decanoyl-, Lauroyl-, Myristoyl-, Palmitoyl- und Stearoylrest ausgewählt ist, Ester der Weinsäure, Zitronensäure oder Bemsteinsäure bzw. der Salze dieser Säuren mit alkylierter Glucose, insbesondere die Produkte mit der INCI-Bezeichnung Disodium Coco-Glucoside Citrate, Sodium Coco-Glucoside Tartrate und Disodium Coco-Glucoside Sulfosuccinate, Alkylpolyglycolethersulfate und Ethercarbonsäuren mit 8 bis 18 C-Atomen in der Alkylgruppe und bis zu 12 Ethoxygruppen im Molekül, Sulfobemsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Ethoxygruppen.

Weiter bevorzugte anionische Tenside sind Alkylsulfate, Alkylpolyglykolethersulfate und Ethercarbonsäuresalze mit 10 bis 18 C-Atomen in der Alkylgruppe und bis zu 12 Glykolethergruppen im Molekül und Sulfobemsteinsäuremono- und -dialkylester mit 8 bis 18 C-Atomen in der Alkylgruppe und Sulfobemsteinsäuremono-alkylpolyoxyethylester mit 8 bis 18 C-Atomen in der Alkylgruppe und 1 bis 6 Oxyethylgruppen. Besonders bevorzugte anionische Tenside sind die Alkali- oder Ammoniumsalze des Laurylethersulfates mit einem Ethoxylierungsgrad von 2 bis 4 EO.

Insbesondere bevorzugte erfindungsgemäße kosmetische Mittel sind dadurch gekennzeichnet, dass sie - bezogen auf ihr Gewicht - 0,5 bis 20 Gew.-%, vorzugsweise 1,0 bis 17,5 Gew.-% und insbesondere 5 bis 15 Gew.-% anionische(s) Tensid(e), besonders bevorzugt Fettalkoholethersulfate der Formel H₃C-(CH₂)ₙ-(OCH₂CH₂)ₖ-OSO₃⁻ M⁺ enthalten, in der n für Werte von 5 bis 21, vorzugsweise von 7 bis 19, besonders bevorzugt von 9 bis 17 und insbesondere von 11 bis 13 und k für Werte von 1, 2, 3, 4, 5, 6, 7, 8, 9 oder 10, vorzugsweise für 1, 2 oder 3 und insbesondere für 2 stehen, und M für ein Kation aus der Gruppe Na⁺, K⁺ NH₄⁺, ½ Mg²⁺, ½ Zn²⁺, vorzugsweise für Na⁺, stehen.

Bevorzugt ist weiterhin die Verwendung kosmetischer Mittel, die amphotere und/oder zwitterionische Tenside enthalten. Der Gewichtsanteil der amphoteren und/oder zwitterionischen Tenside am Gesamtgewicht der kosmetischen Mittel beträgt vorzugsweise 0,5 bis 50 Gew.-%, bevorzugt 1,0 bis 40 Gew.-% und insbesondere 2,0 bis 30 Gew.-%, jeweils bezogen auf das Gesamtgewicht Mittel.

Als zwitterionische Tenside und Emulgatoren werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside und Emulgatoren sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamidderivat.

Unter ampholytischen Tensiden und Emulgatoren werden solche oberflächenaktiven Verbindungen verstanden, die außer einer C₈ - C₂₄ - Alkyl- oder -Acylgruppe mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Besonders bevorzugte erfindungsgemäße kosmetische Mittel sind dadurch gekennzeichnet, dass sie amphotere(s) Tensid(e) aus den Gruppen der
- N-Alkylglycine,
- N-Alkylpropionsäuren,
- N-Alkylaminobuttersäuren,
- N-Alkyliminodipropionsäuren,
- N-Hydroxyethyl-N-alkylamidopropylglycine,
- N-Alkyltaurine,
- N-Alkylsarcosine,
- 2-Alkylaminopropionsäuren mit jeweils etwa 8 bis 24 C-Atomen in der Alkylgruppe,
- Alkylaminoessigsäuren mit jeweils etwa 8 bis 24 C-Atomen in der Alkylgruppe,
- N-Kokosalkylaminopropionat,
- Kokosacylaminoethylaminopropionat
- C₁₂ - C₁₈ - Acylsarcosin,
- N-Alkyl-N,N-dimethylammonium-glycinate, beispielsweise Kokosalkyl-dimethylammoniumglycinat,
- N-Acyl-aminopropyl-N,N-dimethylammoniumglycinate, beispielsweise Kokosacylaminopropyl-dimethylammoniumglycinat,
- 2-Alkyl-3-carboxymethyl-3-hydroxyethyl-imidazoline mit jeweils 8 bis 18 C-Atomen in der Alkyl- oder Acylgruppe
- Kokosacylaminoethylhydroxyethylcarboxymethylglycinat
- der unter der INCI-Bezeichnung Cocamidopropyl Betain bekannten Verbindungen,
- der unter der INCI-Bezeichnung Disodium Cocoamphodiacetate bekannten Verbindungen
enthalten, wobei bevorzugte Haarbehandlungsmittel dadurch gekennzeichnet sind, dass sie 5 bis 30 Gew.-%, vorzugsweise 6 bis 25 Gew.-%, weiter bevorzugt 7 bis 20 Gew.-%, noch weiter bevorzugt 8 bis 15 Gew.-% und insbesondere 10 bis 12,5 Gew.-% amphotere(s) Tensid(e) enthalten.

Besonders bevorzugte Mittel enthalten als amphotere Tenside Betaine der Formel (Bet-I) 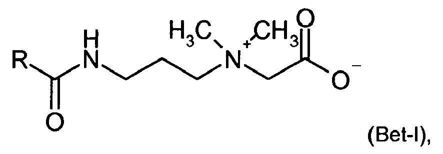 in der R für einen geradkettigen oder verzweigten, gesättigten oder ein- bzw. mehrfach ungesättigten Alkyl- oder Alkenylrest mit 8 bis 24 Kohlenstoffatomen steht. Besonders bevorzugte Haarbehandlungsmittel sind dadurch gekennzeichnet, dass sie - bezogen auf ihr Gewicht - 0,25 bis 8 Gew.-%, weiter bevorzugt 0,5 bis 7 Gew.-%, weiter bevorzugt 0,75 bis 6,5 Gew.-% und insbesondere 1 bis 5,5 Gew.-% Tensid(e) der Formel (Bet-I) enthalten, in der R für einen geradkettigen oder verzweigten, gesättigten oder ein- bzw. mehrfach ungesättigten Alkyl- oder Alkenylrest mit 8 bis 24 Kohlenstoffatomen steht.

Diese Tenside werden nach der INCI-Nomenklatur als Amidopropylbetaine bezeichnet, wobei die Vertreter, die sich von Kokosfettsäuren ableiten, bevorzugt sind und als Cocoamidopropylbetaine bezeichnet werden. Besonders bevorzugt werden erfindungsgemäß Tenside der Formel (Bet-I) eingesetzt, die ein Gemisch der folgenden Vertreter sind:
H₃C-(CH₂)₇-C(O)-NH-(CH₂)₃N⁺(CH₃)₂CH₂COO⁻
H₃C-(CH₂)₉-C(O)-NH-(CH₂)₃N⁺(CH₃)₂CH₂COO⁻
H₃C-(CH₂)₁₁-C(O)-NH-(CH₂)₃N⁺(CH₃)₂CH₂COO⁻
H₃C-(CH₂)₁₃-C(O)-NH-(CH₂)₃N+(CH₃)₂CH₂COO⁻
H₃C-(CH₂)₁₅-C(O)-NH-(CH₂)₃N⁺(CH₃)₂CH₂COO⁻
H₃C-(CH₂)₇-CH=CH-(CH₂)₇-C(O)-NH-(CH₂)₃N⁺(CH₃)₂CH₂COO⁻

Besonders bevorzugt werden Tenside der Formel (Bet-I) innerhalb engerer Mengenbereiche eingesetzt. Hier sind erfindungsgemäße Mittel bevorzugt, die - bezogen auf ihr Gewicht - 0,25 bis 8 Gew.-%, weiter bevorzugt 0,5 bis 7 Gew.-%, weiter bevorzugt 0,75 bis 6,5 Gew.% und insbesondere 1 bis 5,5 Gew.-% Tensid(e) der Formel (Bet-I) enthalten.

Zusätzlich zu dem bzw. den Amphotensiden der Formel (Bet-I) oder an deren Stelle können die erfindungsgemäßen Kosmetische Mittel mit besonderem Vorzug als amphotere Tenside Betaine der Formel (Bet-II) 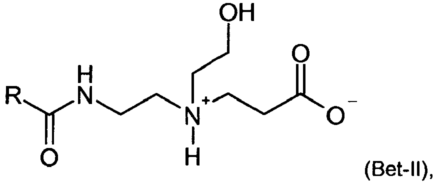 enthalten, in der R für einen geradkettigen oder verzweigten, gesättigten oder ein- bzw. mehrfach ungesättigten Alkyl- oder Alkenylrest mit 8 bis 24 Kohlenstoffatomen steht.
Besonders bevorzugte Haarbehandlungsmittel sind dadurch gekennzeichnet, dass sie - bezogen auf ihr Gewicht - 0,25 bis 8 Gew.-%, weiter bevorzugt 0,5 bis 7 Gew.-%, weiter bevorzugt 0,75 bis 6,5 Gew.-% und insbesondere 1 bis 5,5 Gew.-% Tensid(e) der Formel (Bet-II) enthalten 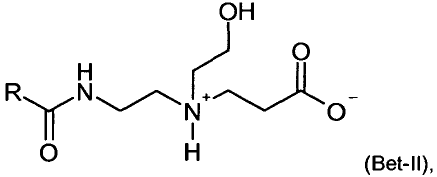 in der R für einen geradkettigen oder verzweigten, gesättigten oder ein- bzw. mehrfach ungesättigten Alkyl- oder Alkenylrest mit 8 bis 24 Kohlenstoffatomen und M für ein Kation steht.

Diese Tenside werden nach der INCI-Nomenklatur als Amphoacetate bezeichnet, wobei die Vertreter, die sich von Kokosfettsäuren ableiten bevorzugt sind und als Cocoamphoactetate bezeichnet werden.

Aus herstellungstechnischen Gründen enthalten Tenside dieses Typs immer auch Betaine der Formel (Bet-IIa) 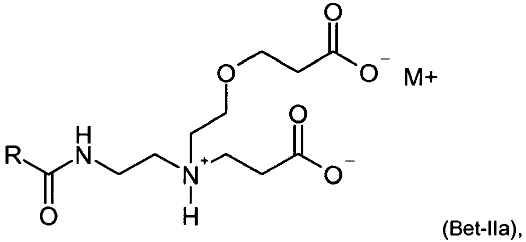 in der R für einen geradkettigen oder verzweigten, gesättigten oder ein- bzw. mehrfach ungesättigten Alkyl- oder Alkenylrest mit 8 bis 24 Kohlenstoffatomen und M für ein Kation steht.

Diese Tenside werden nach der INCI-Nomenklatur als Amphodiacetate bezeichnet, wobei die Vertreter, die sich von Kokosfettsäuren ableiten, bevorzugt sind und als Cocoamphodiactetate bezeichnet werden.

Besonders bevorzugt werden erfindungsgemäß Tenside der Formel (Bet-II) eingesetzt, die ein Gemisch der folgenden Vertreter sind:
H₃C-(CH₂)₇-C(O)-NH-(CH₂)₂NH⁺(CH₂CH₂OH)CH₂CH₂COO⁻
H₃C-(CH₂)₉-C(O)-NH-(CH₂₎₂NH⁺(CH₂CH₂OH)CH₂CH₂COO⁻
H₃C-(CH₂)₁₁-C(O)-NH-(CH₂)₂NH⁺(CH₂CH₂OH)CH₂CH₂COO⁻
H₃C-(CH₂)₁₃-C(O)-NH-(CH₂)₂NH⁺(CH₂CH₂OH)CH₂CH₂COO⁻
H₃C-(CH₂)₁₅-C(O)-NH-(CH₂)₂NH⁺(CH₂CH₂OH)CH₂CH₂COO⁻
H₃C-(CH₂)₇-CH=CH-(CH₂)₇-C(O)-NH-(CH₂)₂NH⁺(CH₂CH₂OH)CH₂CH₂COO⁻

Besonders bevorzugt werden Tenside der Formel (Bet-II) innerhalb engerer Mengenbereiche eingesetzt. Hier sind erfindungsgemäße Mittel bevorzugt, die - bezogen auf ihr Gewicht - 0,25 bis 8 Gew.-%, weiter bevorzugt 0,5 bis 7 Gew.-%, weiter bevorzugt 0,75 bis 6,5 Gew.-% und insbesondere 1 bis 5,5 Gew.-% Tensid(e) der Formel (Bet-II) enthalten.

Zusammenfassend sind erfindungsgemäße Kosmetische Mittel bevorzugt, bei denen der Rest R in den Formeln (Bet-I) und (Bet-II) ausgewählt ist aus
- H₃C-(CH₂)₇-
- H₃C-(CH₂)₉-
- H₃C-(CH₂)₁₁-
- H₃C-(CH₂)₁₃-
- H₃C-(CH₂)₁₅-
- H₃C-(CH₂)₇-CH=CH-(CH₂)₇-
oder Mischungen aus diesen.

Zusätzlich zu den anionischen und amphoteren/zwitterionischen Tensiden können die erfindungsgemäß verwendeten Mittel auch nichtionische Tenside enthalten.

Besonders bevorzugte nichtionische Tenside sind Alkylpolyglycoside. Demnach sind erfindungsgemäße Kosmetische Mittel bevorzugt, die als nichtionische Tenside - bezogen auf ihr Gewicht - 0,1 bis 20 Gew.% Alkylpolyglycoside der allgemeinen Formel RO-(Z)ₓ enthalten, wobei R für Alkyl, Z für Zucker sowie x für die Anzahl der Zuckereinheiten steht.

Alkylpolyglycoside (APG) sind nichtionische Tenside, die vollständig aus nachwachsenden Rohstoffen (Zuckerbausteine, vorwiegend Glucose z.B. aus Maisstärke und Fettalkohol z.B. aus Kokosöl) hergestellt werden. Alkylpolyglycoside sind durch sauer katalysierte Reaktion (Fischer-Reaktion) von Zuckern, insbesondere Glucose (oder Stärke) oder von Butylglycosiden mit Fettalkoholen zugänglich. Dabei entstehen komplexe Gemische aus Alkylmonoglucosid (Alkyl-α-D- und -β-D-glucopyranosid sowie geringe Anteile -glucofuranosid), Alkyldiglucosiden (-isomaltoside, -maltoside etc.) und Alkyloligoglucosiden (-maltotrioside, -tetraoside etc.). Der durchschnittliche Polymerisationsgrad kommerzieller Produkte, deren Alkyl-Reste im Bereich C8-C16 liegen, beträgt 1,2-1,5.

Erfindungsgemäß bevorzugt werden Alkylpolyglycoside entsprechend der allgemeinen Formel RO-(Z)ₓ eingesetzt, wobei R für Alkyl, Z für Zucker sowie x für die Anzahl der Zuckereinheiten steht. Besonders bevorzugt sind solche Alkylpolyglycoside, bei denen R
- im Wesentlichen aus C₈- und C₁₀-Alkylgruppen,
- im Wesentlichen aus C₁₂- und C₁₄-Alkylgruppen,
- im Wesentlichen aus C₈- bis C₁₆-Alkylgruppen oder
- im Wesentlichen aus C₁₂- bis C₁₆-Alkylgruppen oder
- im Wesentlichen aus C₁₆ bis C₁₈-Alkylgruppen
besteht.
Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglycoside enthalten im Schnitt 1,1 bis 5 Zuckereinheiten. Alkylpolyglycoside mit x-Werten von 1,1 bis 2,0 sind bevorzugt. Ganz besonders bevorzugt sind Alkylglycoside, bei denen x 1,1 bis 1,8 beträgt.

Weitere Tenside, die - insbesondere in Mischung mit Alkylpolyglycosiden - besonders vorteilhaft in den erfindungsgemäßen Mitteln eingesetzt werden können, sind Glutamate, Asparaginate und Sulfoacetate. Hier sind erfindungsgemäße Kosmetische Mittel bevorzugt, die - bezogen auf ihr Gewicht - 0,1 bis 20 Gew.-% Fettsäureglutamate (Acylglutamate) und/oder Fettsäureasparaginate (Acylasparaginate) und/oder Alkylsulfoacetate (Sulfoessigsäure-alkylester) enthalten.

### Acylglutamate lassen sich durch die Formel

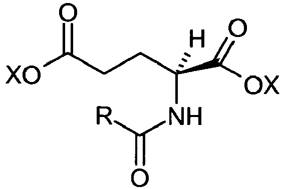

beschreiben, in der R-CO für einen linearen oder verzweigten Acylrest mit 6 bis 22 Kohlenstoffatomen und 0 und/ oder 1, 2 oder 3 Doppelbindungen und X für Wasserstoff, ein Alkali- und/oder Erdalkalimetall, Ammonium, Alkylammonium, Alkanolammonium oder Glucammonium steht.
Überraschenderweise wurde gefunden, dass Abmischungen von Alkylglucosiden mit Acylglutamaten eine sehr gute dermatologische Verträglichkeit und ein verbessertes Schaumvermögen sowohl hinsichtlich des Basisschaums als auch der Schaumstabilität in Gegenwart von Wasserhärte aufweisen.
Acylglutamate stellen bekannte anionische Tenside dar, die beispielsweise durch Schotten-Baumann Acylierung von Glutaminsäure mit Fettsäuren, Fettsäureestem oder -Chloriden erhalten werden können. Verkaufsprodukte sind beispielsweise von der Hoechst AG, Frankfurt/DE oder der Ajinomoto Co. Inc., Tokyo/JP erhältlich.
Typische Beispiele für geeignete Acylglutamate sind Aniontenside, die sich von Fettsäuren mit 6 bis 22, vorzugsweise 12 bis 18 Kohlenstoffatomen ableiten, wie beispielsweise C12/14- bzw. C12/18-Kokosfettsäure, Laurinsäure, Myristinsäure, Palmitinsäure und/oder Stearinsäure. Besonders bevorzugt sind Natrium-N-cocoyl- und Natrium-N-stearoyl-L-glutamat
Die erfindungsgemäßen Mittel können die Alkyl- und/oder Alkenyloligoglucoside und die Acylglutamate im Gewichtsverhältnis 1 : 99 bis 99 : 1, vorzugsweise 10 : 90 bis 90 : 10 und insbesondere 80 : 20 bis 50 : 50 enthalten.
Acylasparaginate lassen sich durch die Formel 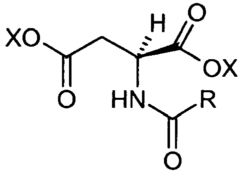 beschreiben, in der R-CO für einen linearen oder verzweigten Acylrest mit 6 bis 22 Kohlenstoffatomen und 0 und/ oder 1, 2 oder 3 Doppelbindungen und X für Wasserstoff, ein Alkali- und/oder Erdalkalimetall, Ammonium, Alkylammonium, Alkanolammonium oder Glucammonium steht.
Acylasparaginate stellen bekannte anionische Tenside dar, die beispielsweise durch Schotten-Baumann Acylierung von Asparaginsäure mit Fettsäuren, Fettsäureestern oder -Chloriden erhalten werden können. Verkaufsprodukte sind beispielsweise von der Hoechst AG, Frankfurt/DE oder der Ajinomoto Co. Inc., Tokyo/JP erhältlich.
Typische Beispiele für geeignete Acylasparaginate sind Aniontenside, die sich von Fettsäuren mit 6 bis 22, vorzugsweise 12 bis 18 Kohlenstoffatomen ableiten, wie beispielsweise C12/14- bzw. C12/18-Kokosfettsäure, Laurinsäure, Myristinsäure, Palmitinsäure und/oder Stearinsäure. Besonders bevorzugt sind Natrium-N-cocoyl- und Natrium-N-stearoyl-L-asparaginat
Die erfindungsgemäßen Mittel können die Alkyl- und/oder Alkenyloligoglucoside und die Acylasparaginate ebenfalls im Gewichtsverhältnis 1 : 99 bis 99 : 1, vorzugsweise 10 : 90 bis 90 : 10 und insbesondere 80 : 20 bis 50 : 50 enthalten.
Sulfoacetate (Sulfoessigsäure-ester), sind üblicherweise Salze von Estern der Sulfoessigsäure und lassen sich durch die allgemeine Formel R-O-C(O)-CH₂-SO₂-OX beschreiben, in der R für einen linearen oder verzweigten Alkyl- oder Alkenylrest mit 6 bis 22 Kohlenstoffatomen und 0 und/ oder 1, 2 oder 3 Doppelbindungen und X für Wasserstoff, ein Alkali- und/oder Erdalkalimetall, Ammonium, Alkylammonium, Alkanolammonium oder Glucammonium steht.
Besonders bevorzugt ist der Einsatz vom Natriumsalz der Sulfoessigsäure mit der INCI-Bezeichnung: Sodium Lauryl Sulfoacetate): H₃C-(CH₂)₁₁-O-CO-CH₂-SO₂-ONa
Natriumlaurylsulfoacetat ist ein weißes, freifließendes Pulver, das neutral reagiert, mit gutem Schaumvermögen, Netzvermögen und Dispergiervermögen.

In einer weiteren bevorzugten Ausführungsform können die erfindungsgemäß verwendeten Mittel Emulgatoren enthalten. Emulgatoren bewirken an der Phasengrenzfläche die Ausbildung von wasser-bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Erfindungsgemäß verwendbare Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- Lineare und verzweigte Fettsäuren mit 8 bis 30 C - Atomen und deren Na-, K-, Ammonium-, Ca-, Mg- und Zn - Salze.

Die erfindungsgemäß verwendeten Mittel enthalten die Emulgatoren bevorzugt in Mengen von 0,1 - 25 Gew.-%, insbesondere 0,5 - 15 Gew.-%, bezogen auf das gesamte Mittel.

Bevorzugt können die erfindungsgemäß verwendeten Mittel mindestens einen nichtionogenen Emulgator mit einem HLB-Wert von 8 bis 18 enthalten. Nichtionogene Emulgatoren mit einem HLB-Wert von 10 - 15 können erfindungsgemäß besonders bevorzugt sein.

Als weiterhin vorteilhaft hat es sich gezeigt, wenn zusätzlich zu dem bzw. den Polymer(en) aus der Gruppe der kationischen und/oder amphoteren Polymere weitere Polymere in den erfindungsgemäßen Mitteln enthalten sind. In einer bevorzugten Ausführungsform werden den erfindungsgemäß verwendeten Mitteln daher weitere Polymere zugesetzt, wobei sich sowohl anionische als auch nichtionische Polymere als wirksam erwiesen haben.

Bei den anionischen Polymeren handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder CoMonomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfonsäure, das beispielsweise unter der Bezeichnung Rheothik^{®}11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.
Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vemetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel^{®}305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvemetzte und vernetzte Polyacrylsäuren. Dabei können Allylether von Pentaerythrit, von Sucrose und von Propylen bevorzugte Vemetzungsagentien sein. Solche Verbindungen sind beispielsweise unter dem Warenzeichen Carbopol^{®} im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze^{®} QM im Handel erhältlich.

Die erfindungsgemäß verwendeten Mittel können in einer weiteren Ausführungsform nichtionogene Polymere (G4) enthalten.

Geeignete nichtionogene Polymere sind beispielsweise:
- Vinylpyrrolidon/Vinylester-Copolymere, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) und Natrosol^{®}-Typen (Hercules) vertrieben werden.
- Stärke und deren Derivate, insbesondere Stärkeether, beispielsweise Structure^{®} XL (National Starch), eine multifunktionelle, salztolerante Stärke;
- Schellack
- Polyvinylpyrrolidone, wie sie beispielsweise unter der Bezeichnung Luviskol^{®} (BASF) vertrieben werden. - Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder Hydroxy-Gruppen enthalten.
- Glycosidisch substituierte Silicone.

Es ist erfindungsgemäß auch möglich, dass die Zubereitungen mehrere, insbesondere zwei verschiedene Polymere gleicher Ladung und/oder jeweils ein ionisches und ein amphoteres und/oder nicht ionisches Polymer enthalten.

Die weiteren Polymere sind in den erfindungsgemäß verwendeten Mitteln bevorzugt in Mengen von 0,05 bis 10 Gew.-%, bezogen auf das gesamte Mittel, enthalten. Mengen von 0,1 bis 5, insbesondere von 0,1 bis 3 Gew.-%, sind besonders bevorzugt.

Zusätzlich kann es sich als vorteilhaft erweisen, wenn in den erfindungsgemäß verwendeten Mitteln Penetrationshilfsstoffe und/ oder Quellmittel enthalten sind. Hierzu sind beispielsweise zu zählen Harnstoff und Hamstoffderivate, Guanidin und dessen Derivate, Arginin und dessen Derivate, Wasserglas, Imidazol und dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol.

Weiterhin kann in einer bevorzugten Ausführungsform der Erfindung ein erfindungsgemäß verwendetes Mittel auch UV - Filter (I) enthalten. Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.
Die erfindungsgemäß verwendeten UV-Filter können beispielsweise ausgewählt werden aus substituierten Benzophenonen, p-Aminobenzoesäureestem, Diphenylacrylsäureestern, Zimtsäureestern, Salicylsäureestern, Benzimidazolen und o-Aminobenzoesäureestern.

Beispiele für erfindungsgemäß verwendbar UV-Filter sind 4-Amino-benzoesäure, N,N,N-Trimethyl-4-(2-oxoborn-3-ylidenmethyl)anilin-methylsulfat, 3,3,5-Trimethyl-cyclohexylsalicylat (Homosalate), 2-Hydroxy-4-methoxy-benzophenon (Benzophenone-3; Uvinul^{®}M 40, Uvasorb^{®}MET, Neo Heliopan^{®}BB, Eusolex^{®}4360), 2-Phenylbenzimidazol-5-sulfonsäure und deren Kalium-, Natrium- und Triethanolaminsalze (Phenylbenzimidazole sulfonic acid; Parsol^{®}HS; Neo Heliopan^{®}Hydro), 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion (Butyl methoxydibenzoylmethane; Parsol^{®}1789, Eusolex^{®}9020), α-(2-Oxobom-3-yliden)-toluol-4-sulfonsäure und deren Salze, ethoxylierte 4-Aminobenzoesäure-ethylester (PEG-25 PABA; Uvinul^{®}P 25), 4-Di-methylaminobenzoesäure-2-ethylhexylester (Octyl Dimethyl PABA; Uvasorb^{®}DMO, Escalol^{®}507, Eusolex^{®}6007), Salicylsäure-2-ethylhexylester (Octyl Salicylat; Escalol^{®}587, Neo Heliopan^{®}OS, Uvinul^{®}018), 4-Methoxyzimtsäure-isopentylester (Isoamyl p-Methoxycinnamate; Neo Heliopan^{®}E 1000), 4-Methoxyzimtsäure-2-ethylhexyl-ester (Octyl Methoxycinnamate; Parsol^{®}MCX, Escalol^{®}557, Neo Heliopan^{®}AV), 2-Hydroxy-4-methoxybenzophenon-5-sulfonsäure und deren Natriumsalz (Benzophenone-4; Uvinul^{®}MS 40; Uvasorb^{®}S 5), 3-(4'-Methylbenzyliden)-D,L-Campher (4-Methylbenzylidene camphor; Parsol^{®}5000, Eusolex^{®}6300), 3-Benzyliden-campher (3-Benzylidene camphor), 4-lsopropylbenzylsalicylat, 2,4,6-Trianilino-(p-carbo-2'-ethylhexyl-1'-oxi)-1,3,5-triazin,3-Imidazol-4-yl-acrylsäure und deren Ethylester, Polymere des N-{(2 und 4)-[2-oxobom-3-ylidenmethyl]benzyl}-acrylamids, 2,4-Dihydroxybenzophenon (Benzophenone-1; Uvasorb^{®}20 H, Uvinul^{®}400), 1,1'-Diphenylacrylonitrilsäure-2-ethylhexyl-ester (Octocrylene; Eusolex^{®}OCR, Neo Heliopan^{®}Type 303, Uvinul^{®}N 539 SG), o-Aminobenzoesäure-menthylester (Menthyl Anthranilate; Neo Heliopan^{®}MA), 2,2',4,4'-Tetrahydroxybenzophenon (Benzophenone-2; Uvinul^{®}D-50), 2,2'-Dihydroxy-4,4'-dimethoxybenzophenon (Benzophenone-6), 2,2'-Dihydroxy-4,4'-dimethoxybenzophenon-5-natri-umsulfonat und 2-Cyano-3,3-diphenylacrylsäure-2'-ethylhexylester. Bevorzugt sind 4-Aminobenzoesäure, N,N,N-Trimethyl-4-(2-oxoborn-3-ylidenmethyl)anilin-methylsulfat, 3,3,5-Trimethylcyclohexylsalicylat, 2-Hydroxy-4-methoxy-benzophenon, 2-Phenylbenzimidazol-5-sulfonsäure und deren Kalium-, Natrium- und Triethanolaminsalze, 3,3'-(1,4-Phenylendimethylenrbis(7,7-dimethyl-2-oxo-bicyclo-[2.2.1]hept-1-yl-methan-sulfonsäure) und deren Salze, 1-(4-tert.-Butylphenyl)-3-(4-me-thoxyphenyl)-propan-1,3-dion, α-(2-Oxoborn-3-yliden)-toluol-4-sulfonsäure und deren Salze, ethoxylierte 4-Aminobenzoesäure-ethylester, 4-Dimethylaminobenzoesäure-2-ethylhexylester, Salicylsäure-2-ethylhexylester, 4-Methoxyzimtsäure-isopentylester, 4-Methoxyzimtsäure-2-ethylhexyl-ester, 2-Hydroxy-4-methoxybenzophenon-5-sulfonsäure und deren Natriumsalz, 3-(4'-Methyl-benzyliden)-D,L-Campher, 3-Benzyliden-campher, 4-lsopropylbenzylsalicylat, 2,4,6-Trianilino-(p-carbo-2'-ethylhexyl-1'-oxi)-1,3,5-triazin, 3-lmidazol-4-yl-acrylsäure und deren Ethylester, Polymere des N-{(2 und 4}-[2-oxoborn-3-ylidenmethyl]benzyl}-acrylamid. Erfindungsgemäß ganz besonders bevorzugt sind 2-Hydroxy-4-methoxy-benzophenon, 2-Phenylbenzimidazol-5-sulfonsäure und deren Kalium-, Natrium- und Triethanolaminsalze, 1-(4-tert.-Butylphenyl)-3-(4-methoxyphenyl)-propan-1,3-dion, 4-Methoxyzimtsäure-2-ethylhexyl-ester und 3-(4'-Methylbenzyliden)-D,L-Campher.

Bevorzugt sind solche UV-Filter, deren molarer Extinktionskoeffizient am Absorptionsmaximum oberhalb von 15 000, insbesondere oberhalb von 20000, liegt.

Weiterhin wurde gefunden, dass bei strukturell ähnlichen UV-Filtern in vielen Fällen die wasserunlösliche Verbindung im Rahmen der erfindungsgemäßen Lehre die höhere Wirkung gegenüber solchen wasserlöslichen Verbindungen aufweist, die sich von ihr durch eine oder mehrere zusätzlich ionische Gruppen unterscheiden. Als wasserunlöslich sind im Rahmen der Erfindung solche UV-Filter zu verstehen, die sich bei 20 °C zu nicht mehr als 1 Gew.-%, insbesondere zu nicht mehr als 0,1 Gew.-%, in Wasser lösen. Weiterhin sollten diese Verbindungen in üblichen kosmetischen Ölkomponenten bei Raumtemperatur zu mindestens 0,1, insbesondere zu mindestens 1 Gew.-% löslich sein). Die Verwendung wasserunlöslicher UV-Filter kann daher erfindungsgemäß bevorzugt sein.

Aus ästhetischen Gründen werden "klare" Produkte von Verbrauchern oft bevorzugt. Erfindungsgemäß bevorzugt verwendete kosmetische Mittel sind daher dadurch gekennzeichnet, dass sie transparent bzw. transluzent sind.

Unter transparent oder transluzent wird im Rahmen der vorliegenden Erfindung eine Zusammensetzung verstanden, die einen NTU-Wert von unter 100 aufweist. Der NTU-Wert (Nephelometric Turbidity Unit, *Nephelometrischer Trübungswert*; NTU) ist eine in der Wasseraufbereitung verwendete Einheit für Trübungsmessungen in Flüssigkeiten. Sie ist die Einheit einer mit einem kalibriertem Nephelometer gemessenen Trübung einer Flüssigkeit.

### Wirkungsnachweise

### Beispiel 1: Nachweis der differentiellen Expression von haarrelevanten Genen

Haarkeratine bilden den quantitativ größten Anteil der Vielzahl der haarschaftbildenden Strukturproteine und gewährleisten die Festigkeit der Haare.

Die Expression verschiedener Haarkeratine im organotypischen Modell kann mit Hilfe eines quantitativen Real-Time-PCR-Verfahrens untersucht werden. Zur Durchführung der PCR wird zunächst mit Hilfe des RNeasy Mini Kits der Firma Qiagen die RNA aus den organotypischen Modellen isoliert und mittels reverser Transkription in cDNA umgeschrieben. Bei der anschließenden PCR Reaktion, die mit Hilfe genspezifischer Primer für die jeweiligen Haarkeratine durchgeführt wird und die der Amplifikation der gesuchten Genabschnitte dient, wird die Bildung der PCR-Produkte online über ein Fluoreszenzsignal detektiert. Das Fluoreszenzsignal ist dabei proportional zur Menge des gebildeten PCR-Produktes. Je stärker die Expression eines bestimmten Gens ist, desto größer ist die Menge an gebildetem PCR-Produkt und umso höher ist das Fluoreszenzsignal.
Zur Quantifizierung des Genexpression wird die unbehandelte Kontrolle gleich 1 gesetzt und die Expression der zu bestimmenden Gene darauf bezogen (x-fache Expresiion). Dabei werden Werte, die größer/gleich der 1,5-fachen Expression der unbehandelten Kontrolle sind, als signifikant eingestuft.

**Tabelle 1: Einfluss von Edelweiß auf die Expression haarrelevanter Gene Expressionsschema verschiedener Haarkeratine nach 24-stündiger Behandlung von Haarfollikelmodellen mit Edelweiß-Extrakt bezogen auf die unbehandelte Kontrolle [=1]**

| | KRT33A | | KRT34 | |
|---|---|---|---|---|
| | Mittelwert | Standardabweichung | Mittelwert | Standardabweichung |
| Kontrolle | 1,0 | 0,0 | 1,0 | 0,1 |
| 0,1 % Edelweiß-Extrakt | 2,0 | 0,0 | 1,5 | 0,1 |
| 1,0 % Edelweiß-Extrakt | 2,4 | 0,1 | 1,6 | 0,1 |

Edelweiß-Extrakt führt zu einer erhöhten Genexpression der untersuchten Markergene. Ausgehend von diesem Expressionsprofil kann auf die Haarstruktur-fördernde Wirkung von Edelweiß rückgeschlossen werden.

### Beispiel 2: Nachweis der Freisetzung von Wachstumsfaktoren

Die beiden Wachstumsfaktoren Hepatocyte Growth Factor (HGF) und Keratinocyte Growth Factor (KGF) sind wichtige Regulatoren des Haarwachstums, die von der dermalen Papille ausgeschüttet werden und an verschiedenen Stellen im Haarfollikel weitere biologische Prozesse auslösen. Bei einer potentiell wachstumsfördemden und das Haar stärkenden Substanz kann von einer Steigerung der in Medium ausgeschütteten Faktoren ausgegangen werden..

Im Speziellen stimuliert HGF das Follikelwachstum und KGF die Keratinozytenproliferation. Entsprechend werden HGF und KGF in der Wachstumsphase vermehrt exprimiert. Darüber hinaus ist HGF maßgeblich an der Regulation des Haarzyklus beteiligt, wobei eine hohe HGF-Konzentration im Haarfollikel in der Phase des aktiven Haarwuchses nachgewiesen wird.

Weitere Untersuchungen zeigen, dass HGF neben der Aktivierung des Haarfollikel-Wachstums auch zu einer signifikanten Verzögerung der Regressionsphase der Haarfollikel führt. Dies wiederum induziert eine signifikante Verlängerung der Haarwachstumsphase und verhindert vorzeitigen Haarausfall.

Die Ausschüttung der Wachstumsfaktoren wurde mit Hilfe des Bioplex - Verfahrens ermittelt. Die Bioplexuntersuchung beruht auf dem Prinzip eines Sandwich - Immunoassays. Ein spezifischer, beadkonjugierter Primärantikörper bindet an das Zielprotein in der Probe, welches wiederum spezifisch von einem fluoreszenzmarkiertem Detektionsantikörper erkannt wird.

Die Untersuchung der Ausschüttung von Wachstumsfaktoren ins Medium erfolgte nach einer Behandlungszeit von 72h. Als Kontrolle wurden unbehandelte Modelle mitgeführt. Alle Untersuchungen erfolgten als Dreifachbestimmung.

**Tabelle 2: Steigerung der HGF und KGF-Freisetzung nach 72-stündiger Behandlung des organotypischen Haarfollikelmodells im Vergleich zur unbehandelten Kontrolle**

| | HGF | | KGF | |
|---|---|---|---|---|
| | Mittelwert | Standardabweichung | Mittelwert | Standardabweichung |
| Kontrolle | 100 | 8 | 100 | 13 |
| 0,1 % Edelweiß-Extrakt | 97 | 2 | 100 | 11 |
| 0,5 % Edelweiß-Extrakt | 140 | 21 | 110 | 15 |
| 1,0 % Edelweiß-Extrakt | 211 | 55 | 133 | 36 |

Die Analysen am Haarfollikelmodell ergaben eine Steigerung der Freisetzung von HGF und KGF um maximal 111 % bzw. 33 %.

## Patentansprüche

1. Verwendung eines Wirkstoffs oder Wirkstoffgemischs, erhältlich aus Pflanzen der Gattung Leontopodium, zur Vitalisierung von Haaren,
wobei die Verwendung eine kosmetische, nicht-therapeutische Verwendung ist, und
wobei die Verwendung topisch erfolgt.

2. Verwendung nach Anspruch 1, **dadurch gekennzeichnet, dass** die Haarfestigkeit erhöht wird.

3. Verwendung nach einem der vorherigen Ansprüche, **dadurch gekennzeichnet, dass** das die Haarstruktur verbessert wird.

4. Verwendung nach einem der vorherigen Ansprüche, dadurch gekenntzeichnet dass das Haarwachstum verbessert wird.

5. Verwendung nach einem der vorherigen Ansprüche, **dadurch gekennzeichnet, dass** die Wirkstoffe aus Pflanzenzubereitungen, insbesondere Presssäften und / oder Extrakten der Pflanzen der Arten Leontopodium alpinum, Leontopodium stracheyi, Leontopodium nivale, Leontopodium palibinianum, Leontopodium souliei, Leontopodium lineariofolium oder Leontopodium gnaphalioides, bevorzugt aus Presssäften und / oder Extrakten der Pflanzen der Arten Leontopodium alpinum, Leontopodium stracheyi und insbesondere bevorzugt aus Presssaften und / oder Extrakten der Pflanzen der Art Leontopodium alpinum gewonnen werden.

6. Verwendung nach einem der vorhergehenden Ansprüche, **dadurch gekennzeichnet, dass** der Wirkstoff oder das Wirkstoffgemisch als Bestandteil eines kosmetischen Mittels eingesetzt wird, welches bezogen auf sein Gesamtgewicht 0,0001 bis 5,0 Gew.-%, vorzugsweise 0,001 bis 4,0 Gew.-% und insbesondere 0,01 bis 3,0 Gew.-% des Wirkstoffs oder des Wirkstoffgemisches enthält.

7. Verwendung nach einem der vorherigen Ansprüche, **dadurch gekennzeichnet, dass** ein kosmetisches Mittel, umfassend
a) einen Wirkstoff oder ein Wirkstoffgemisch, erhältlich aus Pflanzen der Gattung Leontopodium;
b) mindestens einen weiteren von a) verschiedenen Haar pflegenden oder stimulierenden Wirkstoff eingesetzt wird.

8. Verwendung nach einem der Ansprüche 6 oder 7, **dadurch gekennzeichnet, dass** das kosmetische Mittel ein Haarbehandlungsmittel, insbesondere Shampoo, Haarnachspolmittel, Haarget, Haarwasser, Haarkur, Haarcreme, Haarlotion, Haarspray und Haartinktur ist.

## Claims

1. The use of an active ingredient or active ingredient mixture, obtainable from plants of the genus *Leontopodium*, for vitalizing hair, wherein the use is a cosmetic, non-therapeutic use, and wherein the use is topical.

2. The use according to claim 1, **characterized in that** hair strength is increased.

3. The use according to one of the preceding claims, **characterized in that** hair structure is improved.

4. The use according to one of the preceding claims, **characterized in that** hair growth is improved.

5. The use according to one of the preceding claims, **characterized in that** the active ingredients are obtained from plant preparations, in particular pressed juices and/or extracts of plants of the species *Leontopodium alpinum, Leontopodium stracheyi, Leontopodium nivale*, *Leontopodium palibinianum, Leontopodium souliei, Leontopodium lineariofolium* or *Leontopodium gnaphalioides,* preferably from pressed juices and/or extracts of plants of the species *Leontopodium alpinum, Leontopodium stracheyi* and particularly preferably from pressed juices and/or extracts of plants of the species *Leontopodium alpinum.*

6. The use according to one of the preceding claims, **characterized in that** the active ingredient or the active ingredient mixture is used as a component of a cosmetic agent, which, based on its total weight, contains 0.0001 to 5.0 wt.%, preferably 0.001 to 4.0 wt.% and in particular 0.01 to 3.0 wt.% of the active ingredient or of the active ingredient mixture.

7. The use according to one of the preceding claims, **characterized in that** a cosmetic agent is used, comprising
a) an active ingredient or an active ingredient mixture, obtainable from plants of the genus *Leontopodium*;
b) at least one additional active ingredient that nourishes or stimulates hair and that is different from a).

8. The use according to one of claims 6 or 7, **characterized in that** the cosmetic agent is a hair treatment agent, in particular shampoo, hair rinsing agent, hair gel, hair tonic, hair conditioner, hair cream, hair lotion, hair spray and hair tincture.

## Revendications

1. Utilisation d'une substance active ou d'un mélange de substance active, pouvant être obtenu à partir de plantes du genre *Leontopodium*, pour revitaliser les cheveux, l'utilisation étant une utilisation cosmétique non thérapeutique, et l'utilisation est effectuée par voie topique.

2. Utilisation selon la revendication 1, **caractérisée en ce que** la résistance capillaire est accrue.

3. Utilisation selon l'une des revendications précédentes, **caractérisée en ce que** la structure capillaire est améliorée.

4. Utilisation selon l'une des revendications précédentes, **caractérisée en ce que** la croissance des cheveux est améliorée.

5. Utilisation selon l'une des revendications précédentes, **caractérisée en ce que** les substances actives sont obtenues à partir de préparation végétales, en particulier de jus de pressage et/ou d'extraits de plantes des espèces *Leontopodium alpinum, Leontopodium stracheyi, Leontopodium nivale*, *Leontopodium palibinianum, Leontopodium souliei, Leontopodium lineariofolium* ou *Leontopodium gnaphalioides,* de préférence à partir de jus de pressage et/ou d'extraits de plantes des espèces *Leontopodium alpinum, Leontopodium stracheyi* et de façon particulièrement préférée de jus de pressage et/ou d'extraits de plantes de l'espèce *Leontopodium alpinum.*

6. Utilisation selon l'une des revendications précédentes, **caractérisée en ce que** la substance active ou le mélange de substances actives est utilisé comme composant d'une composition cosmétique, qui contient, sur la base du poids total, de 0,0001 à 5,0% en poids, de préférence de 0,001 à 4,0% et en particulier de 0,01 à 3,0% en poids de la substance active ou du mélange de substances actives.

7. Utilisation selon l'une des revendications précédentes, **caractérisée en ce qu'**une composition cosmétique est utilisée qui comprend
a) une substance active ou un mélange de substances actives, qui peut être obtenu à partir de plantes du genre *Leontopodium* ;
b) au moins une autre substance active de soin ou de stimulation capillaire différente de a).

8. Utilisation selon l'une des revendications 6 ou 7, **caractérisée en ce que** la composition capillaire est une composition de traitement cosmétique, notamment un shampooing, une composition de rinçage capillaire, un gel capillaire, une lotion capillaire, un traitement capillaire, une crème capillaire, une lotion capillaire, une laque capillaire, et une teinture capillaire.
